# EUROPEAN PATENT APPLICATION

(11) **EP 4 843 523 A1**
(43) Date of publication of application: **25.11.2026**
(21) Application number: 25177183.8
(22) Date of filing: 19.05.2025
(51) Int. Cl.: A61B 18/14, A61B 18/00

(54) **A FLEXIBLE RADIOFREQUENCY CATHETER AND A RELATED METHOD**

(71) Applicant: F Care Systems, 2600 Antwerpen (BE)
(72) Inventor: DEVERS, Rudi, 2020 Antwerpen (BE)
(74) Representative: Gevers Patents

(57) **Abstract**

The invention provides a method for Perfusion assisted Endoscopic Ultrasound-Guided Radiofrequency Ablation of a target tissue and a related flexible radiofrequency catheter, said flexible radiofrequency catheter comprising a flexible insulated tube that is configured to endoscopically navigate said catheter through an endoscopic channel and an extendable active tip (B) of an electrode which is not insulated that is configured to deliver radiofrequency energy for ablation accurately to said target tissue wherein said extendable active tip further comprises at least one injection hole positioned near said active tip that is configured to inject a liquid solution in said target tissue for altering at least one property of said target tissue for enhancing thermal effects of said energy for ablation in said target tissue.

## Description

### Technical field

The present invention relates to a flexible radiofrequency catheter for perfusion assisted endoscopic ultrasound-guided radiofrequency ablation, and a related method.

### Background art

Pancreatic cancer is one amongst the leading causes of cancer-related deaths, ranking 6^{th}, despite its relatively low incidence rates (3%). While surgery is the management of choice in respectable tumors, 80-85% of pancreatic cancers are diagnosed at an advanced stage and are not amenable to resection. Therefore, there is a need for less invasive alternative therapeutic intervention to avoid short-term and long-term adverse events. Additionally, there is a need for palliative therapies for unresectable and recurrent tumors such as pancreatic tumors.

Currently, Radiofrequency ablation (RFA) has been evolving as an effective minimally invasive alternative to the conventional resection in treating cancer. The principle of operation being focal hyperthermic damage, altering the tumor microenvironment at the membrane and subcellular levels. The ionic agitation due to the radiofrequency increases the local temperatures ranging from 60-120⁰C causing coagulative necrosis and hyperthermic zone.

Endoscopic ultrasound-guided radiofrequency ablation (EUS-RFA) has emerged as a promising technique for the minimally invasive treatment of pancreatic tumors.

Both monopolar and bipolar radiofrequency ablation system are available to be used inside the endoscope for EUSRFA. Monopolar catheters have a closed-loop circuit that includes the radiofrequency generator, a neutral pad and the patient in the circuit. The bipolar catheter has both the active and neutral electrodes interstitially without the need for an external neutral pad.

Such a currently applied flexible radiofrequency catheter for endoscopic ultrasound-guided radiofrequency ablation EUS-RFA of a tumor, such as a pancreatic tumor comprise a flexible insulated tube configured to endoscopically navigate said catheter through an endoscopic channel and a coupled extendable active tip configured to deliver energy for ablation in an accurate manner to a target tissue such as a (pancreatic) tumor, to be ablated. Furthermore, the flexible radiofrequency catheter comprises a handle assembly for controlling tip extension and retraction into the target tissue such as a tumor.

While percutaneous and laparoscopic radiofrequency ablation (RFA) has been effectively used in other organs, their role in treating target tissues such as for example pancreatic tumors is more complicated due to the position of the organ itself. RFA associated risks stem from necrosis of the pancreatic parenchyma and the proximity to critical vascular and pancreatic-biliary ductal structures

### Disclosure of the invention

An object of the present invention is to provide a flexible radiofrequency catheter for perfusion assisted endoscopic ultrasound-guided radiofrequency ablation and a related method, wherein the aforementioned shortcoming or drawbacks of the known solutions are alleviated or overcome.

An additional objective of the present invention is to increase the efficiency and safety of the radiofrequency ablation to a target tissue such as a tumor.

According to the present invention, this objective is achieved by the method as described in the first independent claim and the system as described in the second independent claim.

According to embodiments of the invention, this objective is achieved by the flexible radiofrequency catheter as described in claim 1 and a related method as described in respective claims 13.

Indeed, this objective is achieved by a flexible radiofrequency catheter for endoscopic ultrasound-guided radiofrequency ablation of a target tissue such as tumor(s), like for example, a pancreatic tumor, wherein the flexible radiofrequency catheter comprising a flexible insulated tube that is configured to endoscopically navigate said catheter through an endoscopic channel and whereafter an extendable active tip of an electrode that deliver RF energy for ablation accurately to said target tissue such as a (pancreatic) tumor and before or simultaneous to the application of RF energy for ablation accurately to said target tissue such as a tumor, e.g. a pancreatic tumor, a liquid solution is injected into said target tissue such as a tumor for altering at least one property, such as electrical conductivity, heat retention capacity, vascularity or visibility under imaging techniques of said target tissue, such as a tumor, and as a consequence for enhancing thermal effects of said energy for ablation in said target tissue such as a tumor via at least one injection hole positioned near said active tip of said electrode. This is both valid and effective in a monopolar as well as in a bipolar configuration.

In this manner, by injecting said liquid solution into said target tissue such as a tumor the at least one property, such as electrical conductivity or heat retention capacity or vascularity or visibility under imaging techniques, of said tumor is altered so that thermal effects of said energy for ablation in said target tissue such as a tumor are enhanced, resulting in a more efficient ablation of said target tissue, such as a tumor, resulting in an improved ablation manner with increased safety.

It is furthermore to be noted that different kind of liquid solutions may be injected where each different liquid solution is injected for a different purpose based on a different principle and effect.

Both a saline liquid solution and the sugar liquid solution alter the electrical characteristics of the target tissue such as a tumor.

The sugar liquid solution enhances heat retention capacity of the tissue while saline enhances electrical conductivity. The principle is different.

The motivation of including contrast agent is for visualising the target area under imaging techniques.

The purpose of the injection of embolization agents is the use for adjuvant treatment modality before ablation in order to reduce the tumor vascularity of the target tissue and intraoperative blood loss.

In an embodiment of the present invention, said active tip B of said electrode relates to the monopolar configuration of the radiofrequency catheter according to the present invention.

In another embodiment of the present invention, said active tip B of said electrode additionally comprises an active tip insulation D and neutral electrode part E. The active tip insulation D electrically separates said active tip B from said neutral electrode part E. This relates to the bipolar configuration of the radiofrequency catheter according to the present invention.

In this manner, no additional neutral pad for grounding needs to be applied to patient's lower back or buttocks.

In an embodiment of the present invention, the flexible radiofrequency catheter further comprises a handle assembly for controlling tip extension and retraction in said target tissue such as tumor.

This handles assembly allows to control the distance of said active tip (both in a monopolar or bipolar configuration) extension and retraction in said target tissue such as a tumor.

In still a further embodiment of the present invention, said liquid solution comprises a sugar solution.

The liquid solution to be inserted/injected in the target tissue such as a tumor or the adjacent tissue may be liquid solutions such as monosaccharides (glucose), di saccharide (sucrose) or polysaccharide (starch) solutions applied prior to or simultaneously during radiofrequency ablation, can enhance the ablation effect by modifying tissue conductivity, thereby potentially improving treatment outcomes for target tissues such as pancreatic cancer tissue.

In still a further embodiment of the present invention, said liquid solution comprises a saline solution. The effect of the injection of a saline liquid solution is that the electrical characteristics of the environment, i.e. of the tissue, are altered.

In still a further embodiment of the present invention, said liquid solution comprises a contrast agent or an embolization agent.

The effect of including contrast agent in said liquid solution for injection is an improved visualisation of the target area under imaging techniques.

Further, the purpose of the injection of embolization agents is the use for adjuvant treatment modality before ablation in order to reduce the vascularity of the target tissue tumor and intraoperative blood loss.

Moreover, as mentioned before, in addition to injecting hyperosmotic sugar solutions, the ability of the catheter to perfuse liquid can also be beneficial to embolize or visualize the target area by injecting embolization agent and/or contrast agent before the ablation step.

The combination of radiofrequency ablation RFA with prior vascular embolization has been shown to improve tumor localization, increase ischemia and decrease post procedural lesions, in the pancreatic, liver and renal lesions.

A further embodiment of the present invention relates to a flexible radiofrequency monopolar catheter configuration wherein said active tip of said electrode comprises a biocompatible conductive material such as, but not limited to stainless steel, which is configured to transmit RF energy to said tumor so that the RF energy optimum is reaching the appropriate tissue for ablation.

A further embodiment of the present invention relates to a flexible radiofrequency bipolar catheter model wherein said active tip includes both an active and neutral electrode comprising biocompatible conductive material such as, but not limited to stainless steel, which is configured to transmit RF energy through said target tissue, such as a tumor, to the neutral electrode part, which also comprises a biocompatible conductive material such as, but not limited to stainless steel of said electrode so that the RF energy optimum is reaching the appropriate tissue, i.e. the target tissue for ablation.

The active electrode and neutral electrode are separated by an electrically insulating material such as but not limited to polytetrafluoroethylene.

Still a further embodiment of the present invention relates to a flexible radiofrequency catheter wherein said at least one injection hole is configured to deliver a liquid solution such as, but not limited to, a saline solution, a sugar solution, contrast agents or embolization agents, in said tumor.

In this manner, by before or simultaneous to the application of a RF energy applied to the target tissue such as tumor tissue, applying through said injection holes which are configured to deliver a liquid solution such as, but not limited to a saline solution, a sugar solution, contrast agents or embolization agents, in said target tissue such as tumor, the application of the liquid solution can enhance the ablation effect by modifying tissue conductivity, thereby potentially improving treatment outcomes for pancreatic cancer.

A further embodiment of the present invention relates to a flexible radiofrequency catheter wherein said handle assembly comprises a precision control mechanism with the positioner and handle base for safe and efficient operation.

This embodiment is valid for both the monopolar configuration as the bipolar configuration of the catheter according to the present invention.

Another embodiment of the present invention relates to a flexible radiofrequency catheter wherein said handle assembly further includes an inlet port for the liquid entry into said flexible radiofrequency catheter.

A further embodiment of the present invention relates to a flexible radiofrequency catheter wherein said flexible insulated tube comprises a flexible, biocompatible material, such as, but not limited to polytetrafluoroethylene to navigate safely through said endoscopic channel as such polytetrafluoroethylene flexible insulated tube is **durable, flexible and resistance to corrosion,** rendering such material suitable for both diagnostic and therapeutic applications. Polytetrafluoroethylene, furthermore, is also biocompatible, meaning that it does not cause an adverse response in the body, and is sterilizable.

Another embodiment of the present invention relates to a flexible radiofrequency catheter wherein said handle assembly comprises a positioner and a handle base, and wherein said positioner is configured to set a predetermined length of the active tip of said electrode prior to insertion, and furthermore, said handle base is configured to extend or retract said active tip of said/comprising said electrode, to position and control said tip of said electrode relative to said tumor.

In this manner, the handle base is configured to move the active tip forward according to fixed length wherein this mechanism enhances precision in targeting the target tissue such as tumor while minimizing unintended tissue damage.

### Brief description of the drawings

The invention will be further elucidated by means of the following description and the appended figures.
Figure 1 shows a flexible radiofrequency catheter for Perfusion assisted Endoscopic Ultrasound-Guided Radiofrequency Ablation according to an embodiment of the invention.
Figure 2 shows a *Close-up view of the extendable tip part of the monopolar catheter* according to an embodiment of the present invention.
Figure 3 shows a *close-up view of the extendable tip part of the bipolar catheter* according to an embodiment of the present invention.
Figure 4 shows a catheter handle with inner part according to an embodiment of the present invention.

### Modes for carrying out the invention

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

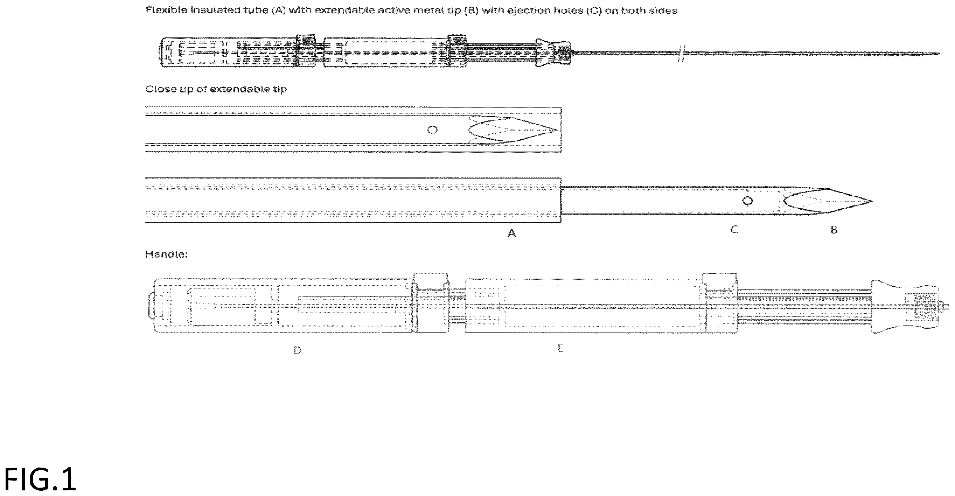

In the following paragraphs, referring to the drawing in FIG.1, an implementation of a flexible radiofrequency catheter for Perfusion assisted Endoscopic Ultrasound-Guided Radiofrequency Ablation of a target tissue according to an embodiment of the present invention is described.

In a further paragraph, all relevant functional means of the flexible radiofrequency catheter as presented in FIG.1 are described followed by a description of all interconnections.

In the succeeding paragraph the actual execution of the Perfusion assisted Endoscopic Ultrasound-Guided Radiofrequency Ablation method of a target tissue by means of the flexible radiofrequency catheter according to an embodiment of the present invention is described.

The flexible radiofrequency catheter for Perfusion assisted Endoscopic Ultrasound-Guided Radiofrequency Ablation of a target tissue first comprises a flexible insulated tube A that is configured to endoscopically navigate said catheter through an endoscopic channel.

The flexible insulated tube of the flexible radiofrequency catheter comprises a flexible, biocompatible material to navigate safely through said endoscopic channel.

The flexible radiofrequency catheter further comprises an extendable active tip B of an electrode which is not insulated and is configured to deliver RF energy for ablation in an accurate manner to said subject target tissue such as a tumor.

The extendable active tip B of the flexible radiofrequency catheter for perfusion further comprises at least one injection hole C that is positioned near said active tip and is configured to inject a liquid solution in said subject target tissue, such as the tumor, for altering at least one property, such as electrical conductivity or heat retention capacity or vascularity or visibility under imaging techniques, of said target tissue for enhancing thermal effects of said energy for ablation in said target tissue.

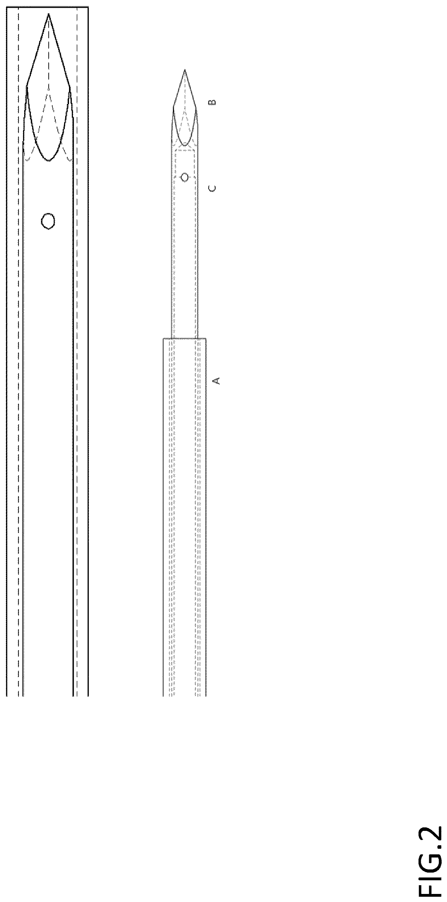

The active tip of said electrode of the flexible radiofrequency catheter comprises a biocompatible conductive material such as but not limited to stainless steel, which is well configured to transmit RF energy to said target tissue such as a tumor. This radiofrequency catheter is further referred to as a monopolar catheter as is shown in Figure 2.

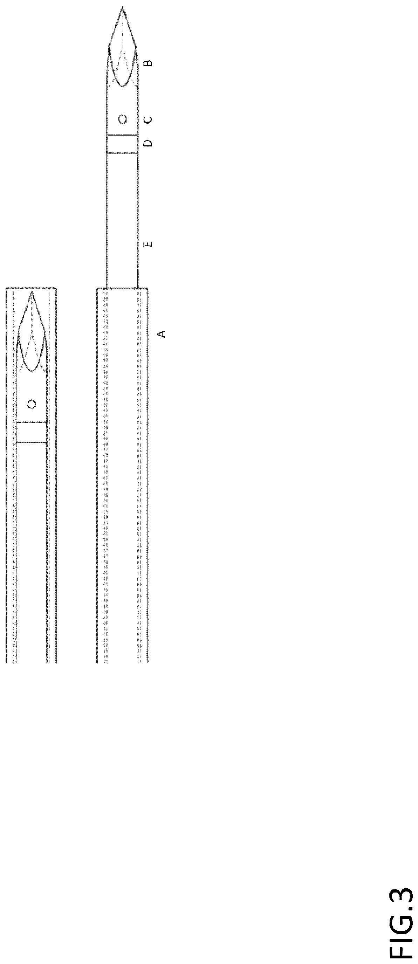

In case of a bipolar catheter, as shown in Fig.3, the active tip B of said electrode additionally comprises an active tip insulation D and neutral electrode part E and the tip insulation part D electrically separating said active electrode tip part B from said neutral electrode part E. In this embodiment of the bipolar catheter the active tip of said electrode comprises an active electrode and neutral electrode part both made of biocompatible conductive material that is optimum configured to transmit **RF** energy from active electrode part B through said target tissue to the neutral electrode part E and wherein said tip insulation is configured to electrically separate said active electrode and said neutral electrode, said tip insulation being made of an isolating material such as polytetrafluoroethylene.

The flexible radiofrequency catheter according to an embodiment of the invention further comprises a handle assembly D, E for controlling a tip extension and retraction in said target tissue.

The handle assembly comprises a precision control mechanism with the positioner and handle base for safe and efficient operation.

The handle assembly of the flexible radiofrequency catheter further includes an inlet port for the liquid entry into said flexible radiofrequency catheter.

The handle assembly further comprises a positioner and a handle base, and wherein the positioner is configured to set a predetermined length of the active tip of said electrode prior to insertion, and said handle base is configured to extend or retract said active tip of said electrode, to position and control said active tip of said electrode relative to said target tissue.

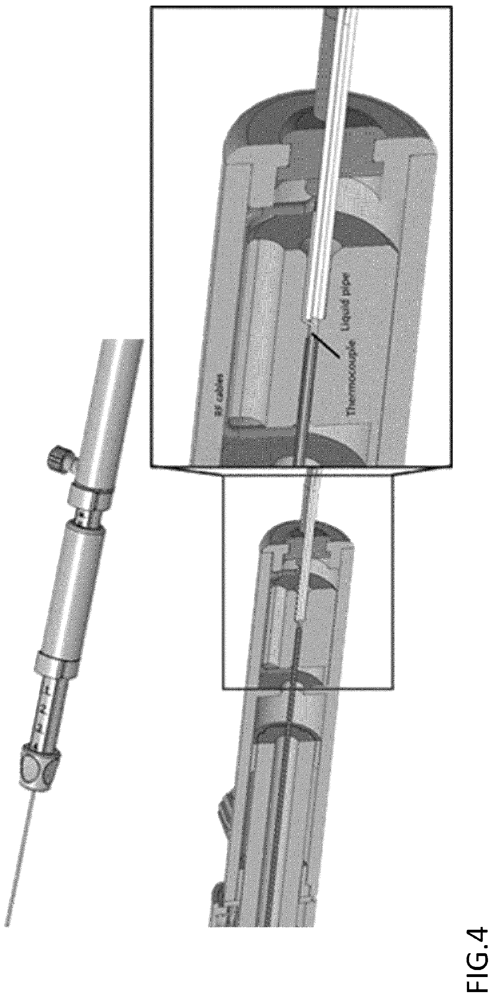

The handle assembly D, E features a user-friendly control system for extending and retracting the metal tip. The positioner is positioned first to fix the length of the active tip. Then the handle base is rotated to move the active tip forward according to fixed length. This mechanism enhances precision in targeting the tissue such as a tumor while minimizing unintended tissue damage. There is a built-in water tubing assembly with inlet in the handle assembly and liquid injection hole (C) near the distal end of the catheter. Thus, the catheter's design allows for improved manoeuvrability and efficiency in EUS-RFA procedures, offering a novel solution for treatment of target tissues such as pancreatic tumor. The 3D drawings of the handle assembly is shown in Figure 4.

In the succeeding paragraph the actual execution of the use of a flexible radiofrequency catheter according to embodiments of the present invention for the Perfusion assisted Endoscopic Ultrasound-Guided Radiofrequency Ablation of a target tissue such as a tumor is explained.

In case of a pancreatic tumor, at first a linear array endoscope is positioned in the stomach in such manner to be in contact with the posterior wall to enable visualization of the pancreas.

Subsequently, the flexible radiofrequency catheter is inserted through an endoscopic channel and by means of adjusting the positioner to optimum setting the length of the active tip based on the lesion size is selected. The active tip of the electrode subsequently is extended by sliding the handle base and advancing the catheter towards the pancreas under Endoscopic Ultrasound guidance (EUS guidance)

The stomach wall is punctured with the active tip of said electrode and the active tip of said electrode is inserted into a target lesion of said target tissue.

A liquid solution is injected into said target tissue e.g. the pancreatic tumor, (including a sugar or starch solution), through said at least one injection hole of said active tip which injecting may take place before or simultaneously to said step of applying **RF** energy at a predetermined power level (using a foot pedal switch) to said target tissue.

It is furthermore to be noted that different kind of liquid solutions may be injected where each different liquid solution is injected for a different purpose based on a different principle and effect.

The insertion of a said liquid solution comprising a sugar or starch solutions in conjunction with EUS-Radiofrequency Ablation for target tissues such as pancreatic cancer tumors enhance the efficacy and safety of the procedure. Sugar solutions such as saccharides such as sucrose or other complex saccharides when present in non-homogenous material such as tissue or tumor, alter the electrical conductivity (reduce the conductivity) of the environment thus enhancing the heating rate of the tissue or tumor. Therefore, the clinical use of sucrose or other saccharides in biological lesions support localized hyperthermia using **RF** energy.

Both a saline liquid solution and the sugar liquid solution alter the electrical characteristics of the environment, i.e. of the tissue.

The sugar liquid solution enhances heat retention capacity of the tissue while saline enhances electrical conductivity. The principle is different.

The motivation of including contrast agent is for visualising the target area under imaging techniques.

The purpose of the injection of embolization agents is the use for adjuvant treatment modality before ablation in order to reduce the vascularity of the target tissue, such as a tumor and intraoperative blood loss.

The Technique involves the use of a flexible radiofrequency catheter for perfusion assisted endoscopic ultrasound-guided radiofrequency ablation (PEUS-RFA) of target tissues such as pancreatic tumors. The monopolar catheter is intended to be used along with the radiofrequency generator, neutral electrodes and pedal switch for activation of **RF** energy. The Bipolar catheter is intended to be used along with the radiofrequency generator and pedal switch for activation of **RF** energy.

The procedure may be performed with two trained operators with the patient positioned appropriately (usually left lateral position) under deep sedation. With the monopolar configuration, the neutral pads are placed on the patients lower back or buttocks.

The feasibility of the procedure was checked by ensuring that it would be possible to insert the catheter and introduce the tip to the target lesion site avoiding the interposed vessels.

This is followed by flushing of the catheter with liquid to be injected at the target site.

Then the catheter is introduced into the endoscope towards the pancreas. The positioner is adjusted to fix the active tip length. The choice of the exposed active tip depends on the size of the lesions to be treated.

While one operator slowly injects the liquid through the catheter, the other operator simultaneously activates the **RF** energy.

The **RF** energy will be applied at a particular preset power setting, for an appropriate duration according to the size of the target lesion. The application of **RF** energy is carried out using manual pressing of the foot pedal switch.

During the activation, the slowly growing hyperechoic zone is monitored carefully using imaging techniques.

The activation and the liquid injection are stopped if the hyper echoic area sufficiently covered the target tissue such as a tumor.

If necessary, the procedure is to be repeated by reinserting the needle in another part of the lesion until obtaining the largest possible ablation of the target tissue such as tumor.

A final remark is that embodiments of the present invention are described above in terms of functional blocks. From the functional description of these blocks, given above, it will be apparent for a person skilled in the art of designing electronic devices how embodiments of these blocks can be manufactured with well-known electronic components. A detailed architecture of the contents of the functional blocks hence is not given.

While the principles of the invention have been described above in connection with specific apparatus, it is to be clearly understood that this description is merely made by way of example and not as a limitation on the scope of the invention, as defined in the appended claims.

## Claims

1. A flexible radiofrequency catheter for Perfusion assisted Endoscopic Ultrasound-Guided Radiofrequency Ablation of a target tissue, said flexible radiofrequency catheter comprising:
a. a flexible insulated tube (A) configured to endoscopically navigate said catheter through an endoscopic channel;
b. an extendable active tip (B) of an electrode configured to deliver radiofrequency energy for ablation accurately to said target tissue, **CHARACTERISED IN THAT** said extendable active tip (B) further comprises:
c. at least one injection hole (C) positioned near said active tip configured to inject a liquid solution in said target tissue for altering at least one property of said target tissue for enhancing thermal effects of said energy for ablation in said target tissue.

2. A flexible radiofrequency catheter according to claim 1, wherein said active tip (B) of said electrode additionally comprises an active tip insulation (D) and neutral electrode part (E), said active tip insulation (D) electrically separating said active tip (B) from said neutral electrode part (E).

3. A flexible radiofrequency catheter according to any of the previous claims, wherein said catheter further comprises a handle assembly for controlling a tip extension and retraction in said target tissue.

4. A flexible radiofrequency catheter according to any of the previous claim, wherein said liquid solution comprises a sugar solution.

5. A flexible radiofrequency catheter according to claim 1 or 3, wherein said liquid comprises a saline solution.

6. A flexible radiofrequency catheter according to claim 1 or 3, wherein said liquid comprises a contrast agent or an embolization agent.

7. A flexible radiofrequency catheter according to claim 1, wherein said active tip of said electrode comprises a biocompatible conductive material, which is configured to transmit radiofrequency energy to said target tissue.

8. A flexible radiofrequency catheter according to claim 2, wherein said active tip of said electrode comprises an active electrode and neutral electrode part both made of biocompatible conductive material, the active electrode configured to transmit radiofrequency energy through said target tissue to the neutral electrode and wherein said tip insulation is configured to electrically separate said active electrode and said neutral electrode, said tip insulation being made of an isolating material.

9. A flexible radiofrequency catheter according to any of the previous claims, wherein said handle assembly (D,E) comprises a precision control mechanism with the positioner and handle base for safe and efficient operation.

10. A flexible radiofrequency catheter according to any of the previous claims, wherein said handle assembly (D,E) further includes an inlet port for the liquid entry into said flexible radiofrequency catheter.

11. A flexible radiofrequency catheter according to any of the previous claims, wherein said flexible insulated tube (A) comprises a flexible, biocompatible material to navigate safely through said endoscopic channel.

12. A flexible radiofrequency catheter according to any of the previous claims, wherein said handle assembly comprises a positioner and a handle base, and wherein:
said positioner is configured to set a predetermined length of the active tip of said electrode prior to insertion, and
said handle base is configured to extend or retract said active tip of said electrode, to position and control said active tip of said electrode relative to said target tissue.

13. Method for performing Perfusion assisted Endoscopic Ultrasound-Guided Radiofrequency Ablation, said method comprising the steps of:
- navigating a flexible insulated tube (A) of said catheter endoscopically through an endoscopic channel;
- delivering, by means of an extendable active tip (B) of an electrode radiofrequency energy for ablation accurately to said target tissue,
**CHARACTERISED IN THAT** said method further comprises the step of:
- injecting, via at least one injection hole (C) positioned near said active tip of said electrode, a liquid in said target tissue for altering at least one property, of said target tissue for enhancing thermal effects of said energy for ablation in said target tissue.

14. A method for performing Perfusion assisted Endoscopic Ultrasound-Guided Radiofrequency Ablation according to claim 13, wherein said method further comprises the steps of:
- positioning a linear array endoscope in the stomach in contact with the posterior wall to enable visualization of the pancreas;
- inserting said flexible radiofrequency catheter through an endoscopic channel and adjusting the positioner to set the length of the active tip based on the lesion size;
- extending said active tip of said electrode by sliding the handle base and advancing the catheter towards the pancreas under EUS guidance;
- puncturing the stomach wall with the active tip of said electrode and inserting the active tip of said electrode into a target lesion of said target tissue,

15. A method for performing Perfusion assisted Endoscopic Ultrasound-Guided Radiofrequency Ablation according to claim 14, wherein said method further comprises the steps of:
- monitoring a development of a hyperechoic zone under ultrasound imaging and ceasing radiofrequency activation and the liquid injection when the zone adequately covers said target tissue; and
- repositioning said active tip of said electrode and repeating the ablation process if necessary to achieve complete target tissue coverage.

16. A method for Perfusion assisted Endoscopic Ultrasound-Guided Radiofrequency Ablation according to any of claims 14 or 16, wherein said liquid solution injected through said at least one injection hole of said flexible radiofrequency catheter to enhance the thermal effect of radiofrequency ablation by altering tissue conductivity or facilitates the effect of ablation.

17. A method for performing endoscopic ultrasound-guided radiofrequency ablation according to any of claims 14 to 17, wherein said injected liquid solution comprises a saccharide solution configured to modulate heating rates and improve ablation efficiency and wherein said method comprises the step of injecting said liquid solution prior or subsequent to said step of applying radiofrequency energy using a radiofrequency generator at a predetermined power level (using a foot pedal switch) to said target tissue.

18. A method for performing endoscopic ultrasound-guided radiofrequency ablation according to any of claims 14 to 18, wherein said injected liquid solution comprises at least one of an embolization agent or cooling liquid, and wherein said method comprises the step of injecting said liquid solution prior or subsequent to said step of applying radiofrequency energy using a radiofrequency generator at a predetermined power level to said target tissue.

19. A method for performing Perfusion assisted Endoscopic Ultrasound-Guided Radiofrequency Ablation according to any of claims 14 to 19, wherein said radiofrequency energy is applied with a neutral electrode pad placed on the patient's lower back or buttocks in case of using a monopolar catheter.

20. A method for performing Perfusion assisted Endoscopic Ultrasound-Guided Radiofrequency Ablation according to any of claims 14 to 20, wherein said ablation process is monitored in real-time using hyperechoic changes observed under endoscopic ultrasound.

21. A method for performing Perfusion assisted Endoscopic Ultrasound-Guided Radiofrequency Ablation according to any of claims 14 to 21, wherein said method is performed by two operators, a first one controlling said injection of said liquid solution and a second one managing said radiofrequency energy application.
